# EUROPEAN PATENT APPLICATION

(11) **EP 1 978 089 A1**
(43) Date of publication of application: **08.10.2008**
(21) Application number: 06834657.6
(22) Date of filing: 14.12.2006
(51) Int. Cl.: C12M 3/00

(54) **CELL CULTURE METHOD AND AUTOMATIC CULTURE SYSTEM USING THE METHOD**

(30) Priority: 17.01.2006 JP 2006008521
(71) Applicant: Hitachi Medical Corporation, Tokyo 101-0021 (JP); National University Corporation Nagoya University, Nagoya-shi, Aichi 464-8603 (JP)
(72) Inventor: SUZUKI, Tsutomu, Tokyo 101-0021 (JP); IEJIMA, Daisuke, Tokyo 101-0021 (JP); KAGAMI, Hideaki, Nagoya-shi Aichi 464-8603 (JP); KATO, Ryuji, Nagoya-shi Aichi 464-8603 (JP); UEDA, Minoru, Nagoya-shi Aichi 464-8603 (JP); OKADA, Kunihiko, Nagoya-shi Aichi 464-8603 (JP)
(74) Representative: Strehl Schübel-Hopf & Partner
(86) International application number: PCT/JP2006/324903
(87) International publication number: WO 2007/083465

(57) **Abstract**

A culturing method according to the present invention including a step of injecting cells extracted from a human body and a culture medium into a culture container, (2) a step of culturing cells while performing air ventilation and exchange of the culture medium in the culture container in which the culture medium and the cells are put, (3) a step of cleaning cultured cells which are cultured in the culture container and then withdrawing the cleaned cells from the culture container into a cell withdrawing vessel while some of the cleaned cells are left in the culture container, (4) a step of injecting a culture medium to the cultured cells left in the culture container in the step (3), and (5) a step of repetitively executing the step (2) and the step (3).

## Description

### Technical Field

The present invention relates to a cell culturing method and a cell culturing apparatus executing the method, and particularly to a cell culturing method and an automatic cell culturing apparatus that can obtain a large amount of cultured cells with preventing contamination of various bacteria, etc. into cultured cells of human bodies.

### Background Art

Recently, many studies concerning regeneration medicine for regenerating damaged tissues, function-declined internal organs, etc. of human bodies have been reported. In the regeneration medicine, a large amount of cells constituting a tissue or internal organ of a human body are required, and cultured cells which are artificially cultured are used as cells required for the regeneration medicine.

As a conventional apparatus relevant to cell culture have been known a cell culturing apparatus in which it is possible to control environments such as the temperature, gas concentration, etc. in a culture chamber as disclosed in Patent Document 1, and an automatic culturing apparatus in which in addition to the control of the environments in a culture chamber, it is also possible to control infusion of a cultured tissue into a culture container, supply/discharge of culture medical agent and further the control of the environments in a culture chamber as disclosed in Patent Document 2.
Patent Document 1: Patent No. 3021789
Patent Document 2: International Publication No. WO2005/059091

In the former conventional culturing apparatus, a work of exchanging culture media in incubators, a successive subculture work for adjusting the cell density to obtain lots of cultured cells, etc. are manually carried out by a researcher or the like. In the case of this apparatus, with respect to the culture media exchanging work, the successive subculture work, etc., after a worker opens the door of the apparatus, he/she lifts up the lid of an incubator such as a petri dish or the like while paying his/her attention so as to prevent contamination of bacteria into the incubator, and carefully perform these works by using a sterilized pipette. When successive subculture is repeated, it is required to perform a work of transplanting multiplied cultured cells to plural incubators at plural times, and thus even a skilled person may induce contamination.

In the latter conventional automatic culturing apparatus of the conventional culturing apparatus, as described above, it is possible to perform the infusion of cells, the supply/discharge of culturing medical agent and further the control of the environment in the culture chamber. However, there is adopted a method of automatically feeding cells cultured in a culturing apparatus out of the apparatus under the state that the cells are poured in a take-out container, and thus it is considered that there is a problem to be solved in contamination when cultured cells are taken out.

When a tissue or internal organ of a human body is regenerated, a large amount of cultured cells are required. However, in order to obtain a large amount of cultured cells, the incubator is required to be designed in large size, and also it is required to increase the amount of cells to be extracted from a human body. However, when the incubator is designed in a large size, the culturing apparatus is necessarily large in size, and thus there occurs a problem that the installation space thereof must be large. Furthermore, when the amount of cells to be extracted from the human body is increased, there occurs a problem that a large damage is applied to the human body.
Patent Document 3 is known as a successive subculture technique of propagating a large amount of experimental cells such as paramecium or the like. According to the technique disclosed in the Patent Document 3, a new culture medium is supplied to cultured cells which are cultured in a culture container containing a fully filled culture medium, and the old culture medium and some of cultured cells are washed away to dilute the cultured cells in the culture container. However, in this technique, the cells which are watched away in the culture medium are directly supplied to measurement, and it seems that this technique is unsuitable to obtain a large amount of pure cells to be supplied to regeneration medicine of human bodies.
Patent Document 3: JP-A-2004-208663

### Disclosure of the Invention

### Problem to be solved by the Invention

An object of the present invention is to provide a cell culturing method and an automatic culturing apparatus that can obtain a large amount of cultured cells used for regeneration medicine of human bodies while responding to the above problem.

### Means of solving the Problem

In order to solve the above problem, a culturing method according to the present invention comprises: (1) a step of injecting cells extracted from a human body and a culture medium into a culture container; (2) a step of culturing cells while performing air ventilation and exchange of the culture medium in the culture container in which the culture medium and the cells are put; (3) a step of cleaning cultured cells which are cultured in the culture container and then withdrawing the cleaned cells from the culture container into a cell withdrawing vessel while some of the cleaned cells are left in the culture container; (4) a step of injecting a culture medium to the cultured cells left in the culture container in the step (3); and (5) a step of repetitively executing the step (2) and the step (4).

In order to solve the above problem, an automatic culturing apparatus of the present invention comprises: a chemical supply unit for individually accommodating plural chemicals necessary for culture; an incubator unit for accommodating a cell culture container; a withdrawing unit for withdrawing cultured cells cultured in the culture container; a supply/discharge unit for supplying the cells to the culture container, supplying the chemicals from the chemical supply unit to the culture container, and discharging cultured cells from the culture container to the withdrawing unit; and a control unit for controlling a chemical supply operation to the culture container, a cell culturing operation in the culture container and a discharge operation of cultured cells from the culture container and has means for making the supply/discharge unit execute, at least once, a cell culturing step containing a chemical supply step of the supply/discharge unit and a cultured cell discharging step on the cultured cells left in the culture container when the cultured cells are withdrawn.

According to the present invention, a large amount of cultured cells used for regeneration medicine of human bodies can be obtained.

### Brief Description of the Drawings

[Fig. 1] Fig. 1 is a block diagram showing a unit construction of an automatic culturing apparatus according to the present invention.
[Fig. 2] Fig. 2 is a block diagram showing the schematic construction of the automatic culturing apparatus according to a first embodiment of the present invention.
[Fig. 3] Fig. 3 is a perspective view showing a culture container in the first embodiment of the present invention.
[Fig. 4] Fig. 4 is a detailed side view of the culture container shown in Fig. 3.
[Fig. 5] Fig. 5 is a cross-sectional view showing a nozzle pipe portion of the culture container.
[Fig. 6] Fig. 6 is a flowchart showing a culturing method of the present invention.
[Fig. 7] Fig. 7 is a block diagram showing the schematic construction of an automatic culturing apparatus according to a second embodiment of the present invention.

### Best Modes for carrying out the Invention

### (First Embodiment)

The present invention will be described hereunder referring to accompanied drawings, and a first embodiment of an automatic culturing apparatus for executing a cell culturing method of the present invention will be first described.

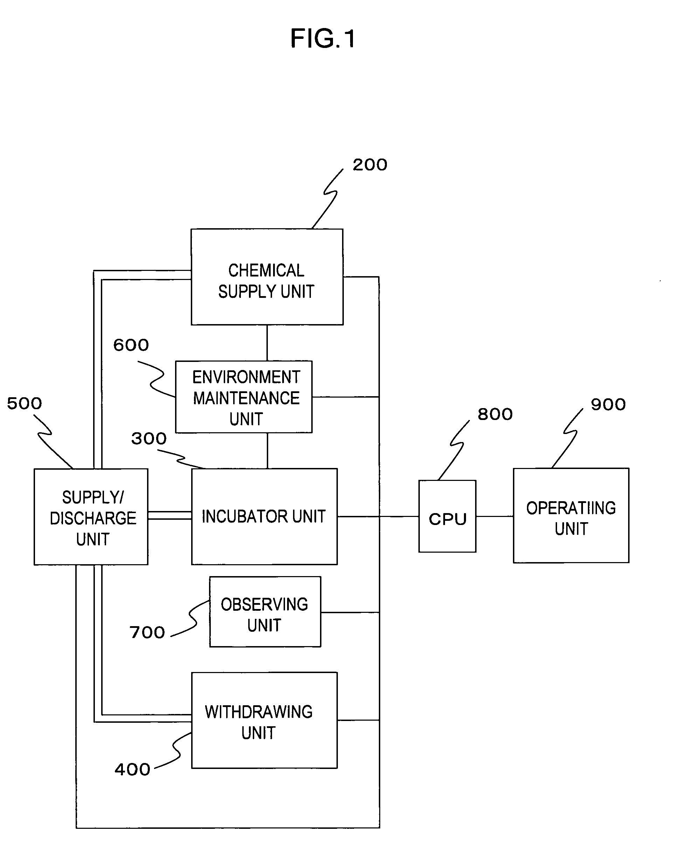

Fig. 1 is a block diagram showing the schematic construction of an automatic culturing apparatus according to a first embodiment of the present invention. In Fig. 1, 200 represents a chemical supply unit for individually accommodating plural chemicals required for cell culture of human cells, and it is constructed so that the inside thereof can be kept in a predetermined temperature range lower than the normal temperature so that the accommodated chemicals are not deteriorated. 300 represents an incubator unit and a culture container is accommodated in the incubator unit. 400 represents a withdrawing unit and withdraws cells cultured in the incubator unit and used chemicals. 500 represents a supply/discharge unit for supplying and discharging chemicals and cells among the chemical supply unit 200, the incubator unit 300 and the withdrawing unit 400. 600 represents an environment maintenance unit and controls the temperature of chemicals supplied from the chemical supply unit 200 to the culture container, the temperature of the incubator unit 300 and the concentration of carbon dioxide (CO₂). 700 represents an observation unit for observing the cell culture state in the culture container accommodated in the incubator unit 30, and it is equipped with a microscope, a moving mechanism for the microscope and illuminating equipment for illuminating the inside of the culture container. 800 represents a central processing unit (CPU) for controlling the withdrawing unit 400, the supply/discharge unit 500, the environment maintenance unit 600 and the observation unit 700. 900 represents an operation unit having an operator for inputting an operation instruction to the automatic culturing apparatus and a monitor for displaying an image obtained by the observation unit 700, etc.

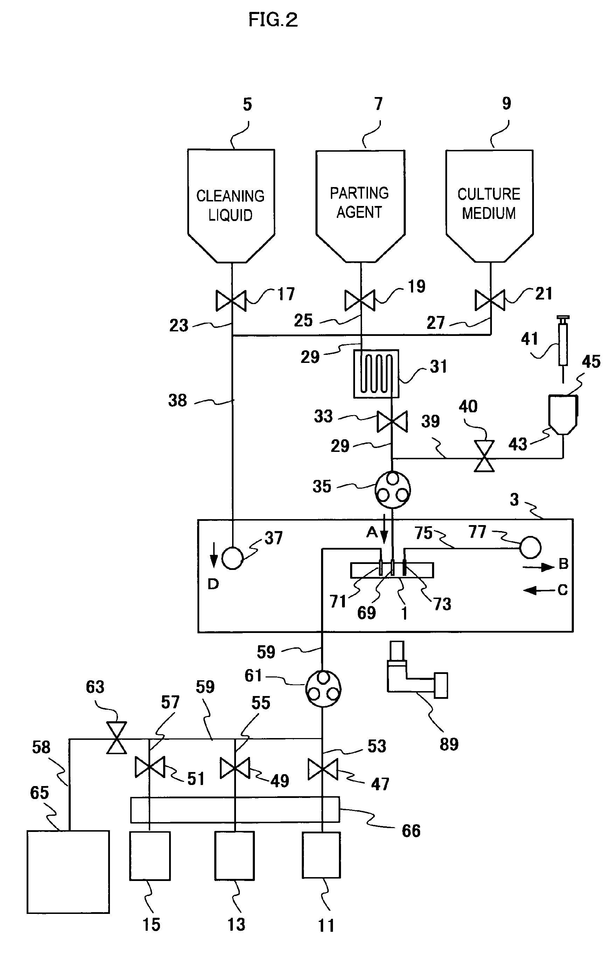

Next, each of the units described above will be described in detail with reference to Fig. 2.
The chemical supply unit 200 has an accommodating box having an openable and closable lid (not shown) and plural (three in the example of Fig. 2) chemical accommodating chemical bags 5, 7, 9 which are disposed at predetermined positions in the accommodating box by a support member. The accommodating box has a heat insulating material for insulating the accommodation space for the chemical bags from the outside and keeping the chemical-bag accommodated space to a predetermined temperature so that the chemicals accommodated in the chemical bags 5, 7, 9 are not deteriorated. It is preferable that the chemical bag 5, 7, 9 is a fluid infusion bag which has foldable flexibility and is formed of material having no toxicity to cells. Specifically, for example, it is preferable to use a bag formed of polyvinyl chloride or silicon rubber. Furthermore, chemicals necessary for culture, for example, cleaning liquid, parting agent and culture medium are individually accommodated in the chemical bags 5, 7 and 9, respectively. The parting agent accommodated in the chemical bag 7 is a chemical required when cells having adhesiveness are culture targets, and it is not required when the culture targets are cells having free property.

The incubator unit 300 is equipped with an incubator 3 as a thermostatic chamber, and a culture container 1 accommodated in the incubator 3. The incubator 3 has an openable and closable lid and the inside thereof can be hermetically sealed by closing the lid as in the case of the chemical supply unit 200. The incubator 3 has an observation window having a bottom surface formed of a transparent material which faces the culture container 1 so that the culture container 1 can be observed from the outside of the incubator 3. The incubator 3 is subjected to thermostatic control so that the inside thereof is kept to a predetermined temperature by a heater described later. In order to enhance the heating efficiency of the heater, it is preferable that the surrounding excluding the observation window is covered by a heat insulating material. The culture container 1 is used to culture cells extracted from a human body, and it is molded by using a material having no toxicity to the cells and translucency, for example polystyrene, polyethylene terephthalate. Furthermore, the inside of the culture container 1 is kept to be hermetically sealed from the external space so that no contamination occurs between cells inserted in the culture container 1 or cultured cells and various bacteria. Therefore, a fixing portion for a tube or the like to be fixed to the culture container 1 is sealed.

The withdrawing unit 400 is equipped with plural (three in this example) cell withdrawing bags 11, 13, 15 for withdrawing cultured cells cultured in the culture container 1, a waste fluid container 65 for withdrawing used chemicals, and a well-known heat sealing mechanism 66 for thermally sealing tube-type injection portions of the cell withdrawing bags 11, 13, 15. It is preferable that a fluid infusion bag which has foldable flexibility and is formed of a material having no toxicity to cells is used as the cell withdrawing bags 11, 13, 15 as in the case of the chemical bags 5, 7, 9. Specifically, for example, a bag of polyvinyl chloride or silicon rubber is preferably used. It is desired to set the size of each of the cell withdrawing bags 11, 13, 15 so that each cell withdrawing bag has an accommodating space in which cells cultured in the culture container 1 by one culturing operation can be accommodated. Furthermore, the number of the cell withdrawing bags equipped in the withdrawing unit 400 is set to three in this example, however, it is desired to set the number of the cell withdrawing bags so that at least the same tissue cells can be sequentially and repetitively cultured in the culture container 1 at plural times.

The supply/discharge unit 500 is equipped with a supply pump 35 for supplying chemicals, cells, air into the culture container 1, a discharge pump 61 for discharging cultured cells and used chemicals from the culture container 1, liquid feeding tubes 23, 25, 27, 29 forming flow passages between the chemical bags 5, 7, 9 and the culture container 1, a gas tube 38 for connecting the liquid feeding tube 29 and a filter 37 described later provided in the incubator 3, a cell injection tube 39 for connecting the liquid feeding tube 29 and a liquid stock container 43, a gas exchange tube 75 for connecting a filter 77 provided in the incubator 3 and the internal space of the culture container 1, and pinch valves 17, 19, 21, 33, 40 provided to the above pipe system. The liquid stock container 43 is used to stock cells which are extracted from a living body and kept to be suspended with culture medium, etc., and has an opening portion covered by a film 45 having flexibility. The film 45 serving as a lid of the liquid stock container 43 is provided to enable a syringe 41 containing the suspended cells to puncture the film 45. Accordingly, in order to prevent various bacteria, etc. from contaminating into the liquid stock container 43, the film 45 is desired to have a predetermined thickness and flexibility so that the puncture hole of the syringe 41 is hermetically sealed.

Furthermore, the supply/discharge unit 500 is equipped with a discharge pump 61 for discharging cultured cells and used chemicals in the culture container 1 , discharge tubes 53, 55, 57, 59 forming flow passages between the culture container 1 and the cell withdrawing bags 11, 13, 15, the waste liquid container 65, and pinch valves 47, 49, 51, 63 provided in the discharge flow passage. In this embodiment, an electromagnetic driving type opening/closing valve is used as each pinch valve so that it can squash the tube having flexibility and close the flow passage. Furthermore, a so-called squeezing pump is used as the supply pump and the discharge pump so that a tube having flexibility is wound around plural rollers disposed in a cylindrical shape and the plural rollers are rotated to press out fluid in the tube.

The environment maintenance unit 600 comprises a heater 31 provided to a part of the liquid feeding tube 29 for feeding chemicals from the chemical bags 19, 21 for accommodating parting agent and culture medium to the culture container 1, a heater, a temperature sensor, a carbon dioxide (CO₂) gas sensor, etc. which are not shown in the figure but provided in the incubator 3. A CO₂ pipe is connected to the incubator 3, and the CO₂ connection pipe is connected to a CO₂ steel cylinder. The heater 31 heats the parting agent and the culture medium in the process of supplying them to the culture container 1. The heater and the temperature sensor provided in the incubator 3 are used to keep the temperature in the culture container 1 to a predetermined temperature. The CO₂ sensor detects the CO₂ concentration in the incubator 3.

The observing unit 700 is disposed at the outside of the observation window of the incubator 3, and it is equipped with a microscope 89 for observing the cell culture state in the culture container 1, an illuminating light source (not shown) provided so as to face the microscope 1 through the culture container 1 and a microscope moving mechanism (not shown) for two-dimensionally moving the microscope 89 in a plane parallel to the bottom surface of the culture container 1. The microscope 89 is constructed to contain a camera which can output pickup image information as an electrical signal, for example, a CCD camera. The microscope moving mechanism may be constructed by disposing moving mechanisms each comprising the combination of a motor and a linear guide mechanism in a cross-arrangement and individually or interlockingly actuating the respective motors, whereby the microscope can be moved to any position.

The central processing unit 800 controls the withdrawing unit 400, the supply/discharge unit 500, the environment maintenance unit 600 and the observing unit 700 according to a culture condition input from an operation unit 900 described later, and it comprises CPU. CPU 800 has ROM, and a software for controlling the operation of the apparatus described later is stored in ROM.

The operation unit 900 is equipped with an operator for inputting an operation (culture) condition of the automatic culture apparatus and a monitor for displaying the operation condition input from the operator and an image picked up by the microscope 89. The pump, the valve, motor, heater and other actuators which are installed in the automatic culture apparatus are driven by a driving circuit, however, these elements are omitted from the figures.

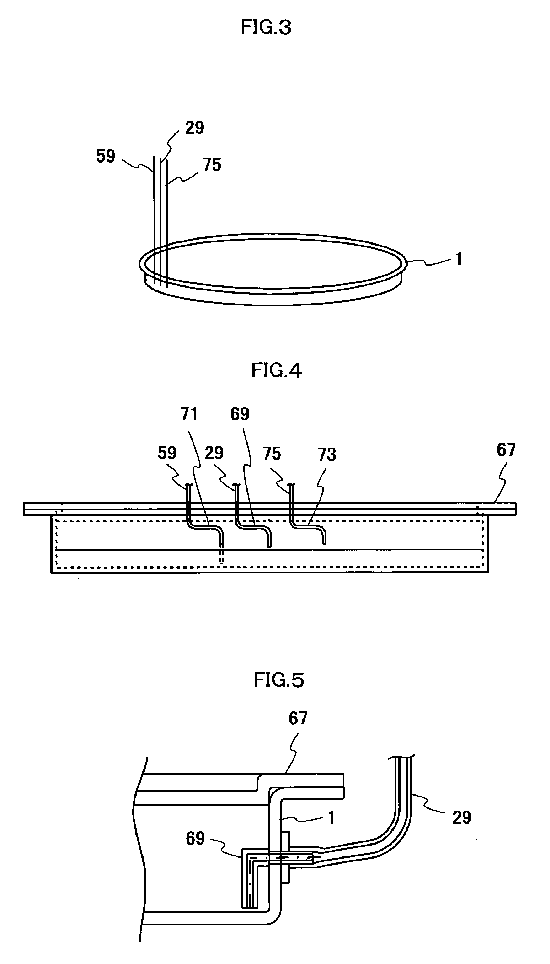

The culture container 1 will be described in detail with reference to Figs. 3 and 4.
The cross-sectional shape of the culture container 1 is not limited to a specific shape, however, in this embodiment, it is a circular petri dish, and a flange is formed at the opening of the upper portion thereof. By using the flat surface of the flange, a lid 67 is fixed by adhesion or the like so that the culture container 1 is hermetically sealed. Nozzles 69, 71, 73 are inserted into the side surface of the culture container 1, and the liquid feeding tube 29 is connected to the nozzle 69, the discharge tube 59 is connected to the nozzle 71 and the gas exchange tube 75 is connected to the nozzle 73. Fig. 5 shows the connection state between the nozzle 69 and the liquid feeding tube 29 in detail.

Next, the positional relationship between the tip of the nozzles 69, 71, 73 and the bottom surface of the culture container 1 will be described. The tip of the nozzle 69 connected to the liquid feeding tube 29 is located in the liquid layer (the liquid level position at the culture time) in the culture container 1 or in the air layer. The tip of the nozzle 71 connected to the discharge tube 59 is set at a position which is spaced from the inner bottom surface of the culture container 1 at a predetermined interval because it is required that some (small amount of) culture-completed cells are left in the culture container 1 for the next culturing operation. Furthermore, the tip of the nozzle 73 connected to the gas exchange tube 75 is set at a higher position than the liquid layer in the culture container 1 because the tip of the nozzle 73 serves as a supply/discharge port for exchanging gas in the culture container 1 and gas in the incubator 3.

Next, the cell culturing method of the present invention will be described in association with the operation of the automatic culturing apparatus described above. The following description will be made by exemplifying a case where culture targets are cells having adhesive property.

### (Preparation of start of culture)

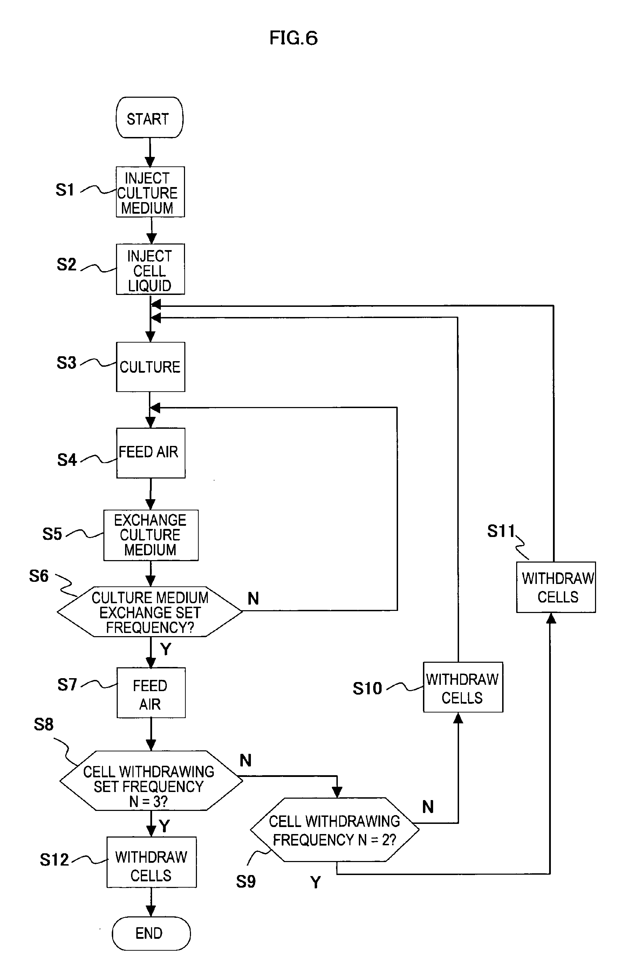

Before culture is started, an operator mounts the cell withdrawing bags 11, 13, 15 in the withdrawing unit 400 of the automatic culture device, and irradiates them with X-ray, γ-ray or the like to subject a sterilization treatment to the inside of the automatic culturing apparatus. The sterile condition of the cell withdrawing bags 11, 13, 15 may be secured by applying radiation ray from the completion of the manufacturing of the bags till the packaging of the bags. After the sterilization treatment is completed, the operator mounts in the chemical supply unit 200 the chemical bags 5, 7, 9 in which the cleaning liquid, the parting agent and the culture medium are stocked. Furthermore, by using a syringe 41, the operator injects into the liquid stock container 43 cell fluid which is obtained by keeping cells, culture medium, etc. under suspension state. After this work is completed, the operator powers on the automatic culturing apparatus, and inputs a culture starting instruction from the operation unit 900. At this time, CPU 800 controls the respective units according to the flowchart of Fig. 6 to execute the cell culturing operation.

### (Step 1: Injection of Culture medium)

When a culture starting instruction is input, CPU 800 first executes the control of injecting culture medium. That is, CPU 800 outputs a signal for opening the pinch valve 21 and the pinch valve 33 of the supply/discharge unit 500, and then outputs a signal for operating the supply pump 35. Accordingly, the flow passages of the liquid feeding tubes 27, 29 are opened, and the supply pump 35 is driven. When the supply pump 35 is driven, the culture medium stocked in the chemical bag 9 is fed through the liquid feeding tubes 27, 29 to the culture container 1. At this time, current is supplied to the heater 31 in response to an instruction of CPU 800, whereby the culture medium is heated in the tube. When a predetermined amount of culture medium is fed to the culture container 1, CPU 800 closes the pinch valve 21 while maintaining the driving of the supply pump 35. At this time, air in the incubator 3 is sucked through the filter 37 into the gas tube 38 and the liquid feeding tube 29. At the timing when the culture medium remaining in the liquid feeding tube 29 is nullified, the supply pump 35 is temporarily stopped in response to an instruction of CPU 800, and the pinch valve 33 is closed again. By feeding air into the liquid feeding tube 29 after the pinch valve 21 is closed, the chemical (culture medium) remaining in the liquid feeding tube 29 is quickly fed into the culture container 1. As a result, the liquid feeding tube 29 is prevented from being clogged with the chemical.

### (Step 2: Cell fluid injection)

After the injection of the culture medium is finished, the injection control of the cell fluid is executed by CPU 800. The cell fluid is stocked in the liquid stock container 43 by the operator as advance preparation, and when an instruction of opening the pinch valve 40 and an instruction of driving the supply pump 35 are output from CPU 800, the pinch valve 40 is opened, and the supply pump 35 is driven, whereby the cell fluid is fed from the liquid stock container 43 through the liquid feeding tube 39 and injected into the culture container 1. After the injection of the cell fluid is finished, the pinch valve 40 is closed and the supply pump 35 is stopped in response to instructions from CPU 800.

### (Step 3: Start of Culture)

When the injection of the cell fluid is finished, CPU 800 cultures cells for a preset period while performing the control of the concentration of CO₂ in the incubator 3 and the control of the temperature in the incubator 3. The preset period is equal to, for example, about three days, however, it may be changed in accordance with the kind of cells, propagation speed of cells or the like.

### (Step 4: Air feeding)

After the above set period elapses, air ventilation in the culture container 1 is carried out. The inside of the culture container 1 of this embodiment is kept substantially hermetically sealed during the cell culturing operation, and it is necessary to carry out air ventilation in the culture container 1 for a proper period. Therefore, CPU 800 outputs an instruction of opening the pinch valve 33 and an instruction of driving the supply pump 35. When the pinch valve 33 is opened and the supply pump 35 is driven, the air in the incubator 3 which is sucked through the filter 37 is supplied through the gas tube 38 and the liquid feeding tube 29 into the culture container 1 (in the direction of an arrow A), and at the same time the air in the culture container 1 is discharged through the gas exchange tube 75 and the filter 77 into the incubator 3 (in the direction of an arrow B). In the flowchart of Fig. 6, the air feeding is carried out once, however, it may be sequentially carried out at any times except for the time of the culture medium exchange operation and the cell withdrawing operation described later. Furthermore, the exchange of air in the culture container 1 may be carried out by supplying air in the incubator 3 from the filter 77 into the culture container 1 (in the direction of an arrow C) and discharging air in the culture container 1 from the filter 37 into the incubator 3 (in the direction of an arrow D).

### (Step 5: Exchange of Culture Medium)

After the air ventilation in the culture container 1 is finished, the exchange of the culture medium is carried out after a predetermined period elapses. The culture medium exchange is periodically carried out to supplement nutrition by discharging the culture medium in the culture container 1 to exclude metabolic substance of cells eluted into the culture medium and injecting new culture medium into the culture container 1. Normally, the operation from the step 3 (S3) of Fig. 6 to the step 5 (S5) is executed every three to seven days. This period may be preset or may be freely changed in accordance with the propagation state of cells. In order to discharge the culture medium, the instruction for opening the pinch valve 63 is output from CPU 800 and also the instruction for driving the discharge pump 61 is output from CPU 800. Accordingly, the pinch valve 63 is opened, the discharge pump 61 is driven, and culture medium containing metabolic substance of cells is discharged from the culture container 1 to the waste liquid container 65 through the discharge tubes 59, 58. When the culture medium is discharged from the culture container 1, the pinch valve 63 is closed, and the exhaust pump 61 is stopped.

When the discharge of the culture medium is completed, a new culture medium is supplied from the chemical bag 9 to the culture container 1. In order to supply the culture medium, CPU 800 outputs an instruction of opening the pinch valves 21, 33, and also outputs an instruction of driving the supply pump 35. Accordingly, the culture medium in the chemical bag 9 is fed through the liquid feeding tubes 27, 29, heated by the heater 31 and then injected into the culture container 1 by only a predetermined amount. When this culture medium exchange is executed, CPU 800 judges whether the frequency of the culture medium exchange reaches a preset frequency. If the culture medium exchange frequency does not reach the preset frequency, the processing returns to step 4 (S4), and the air feeding operation of the step 5 (S5) and the culture medium exchange operation of the step 6 (S6) are repetitively carried out.

### (Step 7: Air Feeding)

When the culture medium exchange frequency reaches the preset frequency, the last air feeding of the step 7 (S4) is carried out as in the case of the step 4 (S4). The management of the CO₂ concentration and the management of the temperature in the incubator 3 are continually performed by the CPU 800 during the cell culturing period between the step 3 (S3) and the step 7 (S7). During the culturing period, the observation unit 700 is properly operated by the operator so that the image of the culture state in the culture container 1 is picked up and a pickup image is displayed on the monitor. The operator observes the image displayed on the monitor to check the proceeding of the culture.

### (Step 8: Judgment of Cell Withdrawing Frequency)

When the air feeding of the step 7 is carried out for a predetermined time, for example, for several days, the withdrawing step of the cultured cells is executed. In the cultured cell withdrawing step, a judgment on the set frequency of the cell withdrawal is first carried out. In this embodiment, the apparatus is set so that cells are withdrawn at three times. In this step 8(S8), if the withdrawal of the cultured cells is judged to be third one, the next step goes to the last cell withdrawing step of the step 13 (S13). If the withdrawal of the cultured cells is judged to be first or second one, the next step goes to step 9 (S9).

### (Step 9: Judgment of Cell Withdrawing Frequency)

In step 8 (S8), it is judged whether the cell withdrawal is second one or not. If the judgment result indicates that the cell withdrawal concerned is not the second one, that is, it is the first one, the next step goes to the cell withdrawing step of step 10 (S10). On the other hand, if the judgment result indicates that the cell withdrawal is the second one, the next step goes to the cell withdrawing step of step 11 (S11).

### (Step 10: First Cell withdrawal)

If the cell withdrawal is judged to be the first one, the discharge step of the culture medium in the culture container 1, the injection step of the cleaning liquid stocked in the chemical bag 5 and the discharge step subsequent to the injection step are carried out, and the cultured cells are cleaned. Thereafter, a step of injecting the parting agent stocked in the chemical bag 7 into the culture container 1 is executed. When a predetermined time elapses from the injection of the parting agent into the culture container 1, the cultured cells are parted from the culture container 1. Subsequently, in connection with the judgment result indicating the first cell withdrawal, a step of withdrawing the cultured cells parted from the bottom surface of the culture container 1 into the cell withdrawing bag 11 is executed.

Therefore, CPU 800 first outputs an instruction of opening the pinch valve 63 and an instruction of driving the discharge pump 61 as in the case of the discharge of the culture medium described with respect to the step 5 (S5), whereby the culture medium in the culture container 1 is discharged. When the discharge of the culture medium is finished, the pinch valve 63 is closed and the discharge pump 61 is stopped on the basis of the instruction from CPU 800. Subsequently, CPU 800 outputs an instruction of opening the pinch valves 17, 33 and outputs an instruction of driving the supply pump 35 to inject the cleaning liquid into the culture container 1. Accordingly, the pinch valves 17, 33 are opened, the supply pump 35 is driven, and the cleaning liquid stocked in the chemical bag 5 is injected into the culture container 1. When a required amount of cleaning liquid is injected into the culture container 1, the supply pump 35 is stopped and the pinch valves 17, 33 are closed. After the cleaning of the cultured cells with the cleaning liquid is finished, the cleaning liquid is fed to the waste liquid container 65 as in the case of the discharge of the culture medium described in step 5. When the cleaning liquid is discharge from the culture container 1, CPU 800 outputs an instruction of opening the pinch valves 19, 33 and an instruction of driving the supply pump 35 to inject the parting agent into the culture container 1. Accordingly, the pinch valves 19, 33 are opened, the supply pump 35 is driven, and the parting agent stocked in the chemical bag 7 is injected into the culture container 1. After the injection of the parting agent is finished, the supply pump 35 is stopped, and the pinch valves 19, 33 are closed. After some time elapses, the cultured cells adhering to the bottom surface of the culture container 1 are floated up by the parting agent. At the timing when the cultured cells are parted from the bottom surface of the culture container 1, CPU 800 injects the culture medium into the culture container 1. The injection of the culture medium at this time is carried out to weaken the efficacy of the parting agent which adversely affects the cells. Therefore, CPU 800 outputs an instruction of opening the pinch valves 21, 33 and an instruction of driving the supply pump 35. Accordingly, the pinch valves 21, 33 are opened, the supply pump 35 is driven, and the culture medium is injected from the chemical bag 9 into the culture container 1. After the injection of the culture medium is finished, the supply pump 35 is stopped, and the pinch valves 21, 33 are closed.

When the culture medium is injected to weaken the efficacy of the parting agent, in exchange of reduction of the adverse effects of the parting agent on the cultured cells, the cultured cells are liable to easily adhere to the culture container 1 again. Accordingly, it is required to quickly withdraw the cultured cells. Therefore, after injecting the culture medium, CPU 800 quickly outputs an instruction of opening the pinch valve (in this case, the pinch valve 47 is opened because of the first withdrawal) and an instruction of driving the discharge pump 61. Accordingly, the cultured cells in the culture container 1 are withdrawn through the discharge tubes 59, 53 into the cell withdrawing bag 11. When the cultured cells are not sucked into the discharge tubes 59, 53, CPU 800 closes the pinch valve 47, and stops the discharge pump 61. Thereafter, CPU 800 outputs an instruction of thermally compressing the tube portion of the cell withdrawing bag 11 to a thermal sealing mechanism 66, whereby the tube portion of the cell withdrawing bag 11 is thermally sealed. Thereafter, the cell withdrawing bag 11 is separated from the apparatus by the operator. The cultured-cell contained bag which is separated from the apparatus is stocked in a cold insulation storage.

Here, a predetermined gap is provided between the tip of the nozzle 71 secured to the culture container 1 and the bottom surface of the culture container 1, so that some of cultured cells are not withdrawn and thus remain in the culture container 1. The subject matter of the present invention resides in that cultured cells which remain or are left in the culture container 1 are repetitively cultured, thereby obtaining a large amount of cultured cells.

### (Second Cell Culture)

Therefore, when the first cell withdrawal of the step 10 (S10) is finished, CPU 800 executes the steps from step 3 (S3) till step 9 (S9) on the cells left in the culture container 1 again, whereby the cells left in the culture container 1 are cultured. CPU 800 judges in step 8 (S8), step 9(S9) how many time is the current cell withdrawal. This judgment result indicates the second cell withdrawal.

### (Step 11: Second Cell Withdrawal)

When the second cell withdrawal is judged in step 9 (S9), in step 11 (S11), the second cell withdrawal is carried out in the same order as the cell withdrawal of the step 10 (S10). The second cell withdrawal in step 11(S11) is different from the step 10 (S10) in that the cells which are discharged from the culture container 1 on the basis of the instruction of CPU 800 in connection with the judgment result of step 9(S9) are withdrawn to the cell withdrawing bag 13, and the thermal sealing mechanism 66 thermally compresses the tube portion of the cell withdrawing bag 13.

### (Third Cell Culture)

When the second cell withdrawal of step 11 (S11) is finished, CPU 800 repetitively executes the steps from step 3(S3) till step 9(S9) on the cells remaining in the culture container 1 again, thereby culturing the cells remaining in the culture container 1. CPU 800 judges in step 8(S8), step 9(S9) how many time is the current cell withdrawal. The judgment result at this time is the third cell withdrawal.

### (Step 12: Third Cell Withdrawal)

When the third cell withdrawal is judged in step 9 (S9), in step 12 (S12), the third cell withdrawal is carried out in the same order as the cell withdrawal of step 10 (S10). However, the third cell withdrawal of step 12 (S12) is different from the step 10 (S10) in that the cells which are discharged from the culture container 1 on the basis of the instruction of CPU 800 in connection with the judgment result of step 9 (S9) are withdrawn into the cell withdrawing bag 15 and the thermal sealing mechanism 62 thermally compresses the tube portion of the cell withdrawing bag 15.

When the third cultured cell withdrawal is finished, the cell withdrawing bags 11, 13, 15 in which the withdrawn cultured cells are accommodated are transferred to a clinical place, and the cultured cells are supplied to tissue regeneration of human bodies.

As described above, according to this embodiment, when the cells cultured in the culture container are withdrawn, some of them are left in the culture container, and the cells left in the culture container are cultured again, whereby a large amount of cells can be proliferated. In this embodiment, the cells left in the culture container are cultured again, and thus a culture container of a small size can be used. As compared with the conventional successive subculture in which cultured cells cultured once in the culture container are transferred to plural different culture containers to continue the cell culture, the apparatus can be more greatly miniaturized. Furthermore, according to this embodiment, tissue cells may be extracted from a living body only once, and thus the damage of the living body can be reduced.

Furthermore, according to this embodiment, cells extracted from a living body are injected into the liquid stock container having the filter by a syringe, and thus the contamination between the cells and the bacteria when the cells are accommodated into the culture container can be prevented. Still furthermore, according to this embodiment, cultured cells are automatically withdrawn into the cell withdrawing bag, and the cell withdrawing bag.is thermally sealed and taken out while the inside thereof is insulated from the external environment. Therefore, the contamination between cells and various bacteria when the cells are withdrawn can be prevented.

According to this embodiment, when chemical is supplied into the culture container, air in the culture container is expelled from the nozzle intercommunicating with the inside of the incubator by the amount corresponding to the amount of the supplied chemical, so that the chemical can be smoothly injected into the culture container. Likewise, when liquid such as chemical or the like is discharged from the culture container, air is supplied from the nozzle intercommunicating with the inside of the incubator into the culture container by the amount corresponding to the discharge amount of liquid containing discharged chemical and cells, and thus the liquid can be smoothly extracted from the culture container.

Furthermore, according to this embodiment, the supply/discharge of air to/from the culture container is carried out through the filter, and thus a clean culture environment is supplied and maintained.

This embodiment has been described by citing the example in which the cell culture and the withdrawal are repetitively carried out totally three times, however, the present invention is not limited to this embodiment. The repetitive frequency of the cell culture and withdrawal may be set to any value in accordance with the required amount of cells insofar as it is equal to at least twice or more. In this case, the number of cell withdrawing bags provided to the withdrawing unit and the number of pinch valves may be changed in accordance with the frequency of the withdrawing cultured cells. If a device for preventing the contamination between the cells and various bacteria in the cell withdrawing operation can be provided, it is unnecessary to mount plural cell withdrawing bags in the automatic culture apparatus in advance, and a method of mounting a cell withdrawing bag in the automatic culture apparatus as occasion demands may be adopted.

In this embodiment, as shown in Fig. 5, the nozzles 69, 71, 73 are provided to the side surface of the culture container, and thus this embodiment is adaptable to a modification in which plural culture containers are stacked and disposed in an incubator.

Furthermore, the culturing apparatus of this embodiment can culture cells substantially continually, and withdraw cells at a predetermined time interval. Accordingly, when a regenerative medical treatment is conducted on an internal organ, a tissue or the like of a patient while dividing the regenerative medical treatment to plural parts, for example when a medical treatment for leukemic disease or the like which subsidiarily uses mesenchymal stem cells is conducted, the mesenchymal stem cells can be supplied over several times at predetermined intervals.

When the cell withdrawing bags 11, 13, 15 and the waste liquid container 65 are connected to the discharge pipe line of one discharge pump 61 through the pinch valves 47, 49, 51, 63 respectively, as in the case of the above embodiment, waste liquid or withdrawn cells may remain in the pipe line. When these waste liquid and dead cells and further metabolic substance of the cells remain in the withdrawing duct, contamination may occur in the next cultured cell withdrawing operation which is repetitively carried out. It is necessary to prevent occurrence of such contamination.

### (Second Embodiment)

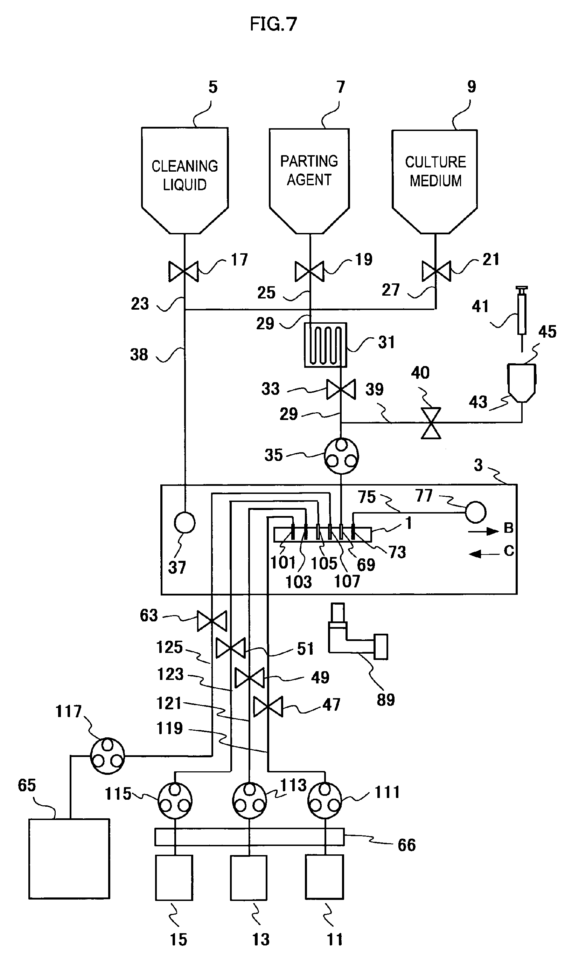

Fig. 7 shows the construction of an automatic culturing apparatus according to a second embodiment which can prevent occurrence of contamination as described above. As shown in Fig. 7, the automatic culturing apparatus of this embodiment is different from the first embodiment in that nozzles 101, 103, 105, 107 corresponding to the number of the cell withdrawing bags 11, 13, 15 and the waste liquid container 65 (totally, four) are provided in place of the nozzle 71 of the discharge system provided to the culture container 1 of the first embodiment. That is, discharge tubes 119, 121, 123, 125 are connected to the nozzles 101, 103, 105, 107. The cell withdrawing bags 11, 13, 15 are connected to the discharge tubes 119, 121, 123, respectively, and the waste liquid container 65 is connected to the discharge tube 125. Furthermore, pinch valves 47, 49, 51 and 63 and discharge pumps 111, 113, 115, 117 are connected to the intermediate positions of these discharge tubes 119, 121, 123, 125, respectively as shown in the drawing. In the drawing, the pinch valves 47, 49, 51, 63 are secured to the suction side of the discharge pumps, however, they may be secured to the discharge side of the discharge pumps. In this embodiment, the discharge pumps 111, 113, 115, 117 and the pinch valves 47, 49, 51, 63 are operated on the basis of instructions of CPU 800.

The thus-constructed automatic culturing apparatus according to this embodiment is different from the automatic culturing apparatus of the first embodiment in the discharge operation of liquid in the culture container 1. That is, when waste liquid in the culture container 1 is discharged, the pinch valve 63 is opened, and the discharge pump 117 is driven. When cells in the culture container 1 are withdrawn, the pinch valve 47 is opened and the discharge pump 111 is driven in the first cell withdrawing operation. Furthermore, in the second cell withdrawing operation, the pinch valve 49 is opened, and the discharge pump 113 is driven. In the third cell withdrawing operation, the pinch valve 51 is opened, and the discharge pump 115 is driven.

According to this embodiment, in addition to the effect of the first embodiment, waste liquid and metabolic substance of cells are prevented from contaminating into cells which are withdrawn in the second and subsequent withdrawing operations because the discharge tubes 119, 121, 123, 125 are provided independently of one another. Accordingly, clean cultured cells can be obtained.

According to the second embodiment, both waste liquid and metabolic substance of cells remaining in the pipe line in the previous cell withdrawing operation are prevented from contaminating into cells which are withdrawn in the second and subsequent cell withdrawing operations, and this embodiment may be modified so that only waste liquid is prevented from contaminating into the cells which are withdrawn in the second and subsequent cell withdrawing operations. This modification may be implemented by independently providing only a withdrawing system of waste liquid to the first embodiment with reference to the second embodiment.

## Claims

1. An automatic culturing apparatus comprising:
a chemical supply unit for individually accommodating plural chemicals necessary for culture;
an incubator unit for accommodating a cell culture container;
a withdrawing unit for withdrawing cultured cells cultured in the culture container;
a supply/discharge unit for supplying the cells to the culture container, supplying the chemicals from the chemical supply unit to the culture container, and discharging cultured cells from the culture container to the withdrawing unit; and
a control unit for controlling a chemical supply operation to the culture container, a cell culturing operation in the culture container and a discharge operation of cultured cells from the culture container and has means for making the supply/discharge unit execute, at least once, a cell culturing step containing a chemical supply step of the supply/discharge unit and a cultured cell discharging step on the cultured cells left in the culture container when the cultured cells are withdrawn.

2. The automatic culturing apparatus according to claim 1, wherein the supply/discharge unit comprises a supply tube pipe for selectively supplying one chemical from plural chemical bags provided to the chemical supply unit to the culture container, a valve provided to the tube pipe and a supply pump.

3. The automatic culturing apparatus according to claim 1, wherein the chemicals accommodated in the plural chemical bags contain culture medium and cleaning liquid.

4. The automatic culturing apparatus according to claim 3, wherein the chemicals accommodated in the plural chemical bags further contain parting agent.

5. The automatic culturing apparatus according to claim 1, wherein the supply/discharge unit comprises plural withdrawing bags for withdrawing cultured cells, a waste liquid container for accommodating used chemicals, a discharging tube pipe for selectively discharging used chemicals and cultured cells from the culture container to the withdrawing bags or the waste liquid container, a valve provided to the tube pipe and a discharge pump.

6. The automatic culturing apparatus according to claim 1, wherein the culture container is provided with plural nozzles connected to the supply/discharge unit.

7. The automatic culturing apparatus according to claim 6, wherein a predetermined gap is provided between the bottom surface of the culture container and the tip of the nozzle connected to the discharging tube pipe of the supply/discharge unit, the tip being located in the culture container, and some of cultured cells are left in the culture container by the gap when the cultured cells are withdrawn.

8. The automatic culturing apparatus according to claim 5, wherein the withdrawing bag is formed of a material having thermo compression property and has a tube portion to be connected to the discharging tube pipe.

9. The automatic culturing apparatus according to claim 8, wherein the supply/discharge unit has a thermal sealing mechanism for thermally compressing the tube portion of a withdrawing bag specified by an instruction from the control unit.

10. The automatic culturing apparatus according to claim 5, wherein the discharging tube pipe of the discharging unit is constructed so that a single tube connected to the culture container is connected to plural tubes connected to the plural withdrawing bags and the waste liquid container, the discharging pump is disposed in the single tube, the valve is disposed in each of the tubes connected to the plural withdrawing bags and the waste liquid container.

11. The automatic culturing apparatus according to claim 5, wherein the discharging tube pipe of the discharging unit has tubes whose number is set to such a value that the culture container is individually connected to each of the waste liquid container and the plural withdrawing bags, and a discharge pump and a valve are disposed in each of the tubes.

12. The automatic culturing apparatus according to claim 5, wherein the discharging tube pipe of the discharging unit comprises a waste liquid tube for connecting the culture container to the waste liquid container, a single tube connected to the culture container and plural tubes for connecting the single tube to the plural withdrawing bags, and a discharging pump and a valve are disposed in each of the waste liquid tube and the single tube connected to the culture container.

13. A cell culturing method comprising:
(1) a step of injecting cells extracted from a human body and a culture medium into a culture container;
(2) a step of culturing cells while performing air ventilation and exchange of the culture medium in the culture container in which the culture medium and the cells are put;
(3) a step of cleaning cultured cells which are cultured in the culture container and then withdrawing the cleaned cells from the culture container into a cell withdrawing vessel while some of the cleaned cells are left in the culture container;
(4) a step of injecting a culture medium to the cultured cells left in the culture container in the step (3); and
(5) a step of repetitively executing the step (2) and the step (3).

14. The cell culturing method according to claim 13, wherein the step (4) and the step (5) are executed at least once.

15. The cell culturing method according to claim 13, wherein the cell withdrawing step in the step (3) contains a step of parting cultured cells from the culture container by injecting parting agent subsequently to a step of cleaning the cultured cells in the culture container.

16. The cell culturing method according to claim 15, further comprising a step of injecting a new culture medium into the culture container to weaken the effect of the parting agent on the cultured cells subsequently to the injection of the parting agent in the step (3).

17. The cell culturing method according to claim 13, wherein the cultured cells withdrawn in the step (5) is withdrawn into a container different from a container in which cells are withdrawn in the step (3).

18. The cell culturing method according to claim 15, wherein the cultured cells withdrawn in the step (5) is withdrawn into a container different from a container in which cells are withdrawn in the step (3).

19. The cell culturing method according to claim 16, wherein the cultured cells withdrawn in the step (5) are withdrawn into a container different from a container in which cells are withdrawn in the step (3).
